# EUROPÄISCHE PATENTANMELDUNG

(11) **EP 2 266 950 A1**
(43) Veröffentlichungstag der Anmeldung: **29.12.2010**
(21) Anmeldenummer: 09008218.1
(22) Anmeldetag: 23.06.2009
(51) Int. Cl.: C07C 235/08, A61K 31/164, A61P 17/00, A61K 8/68, A61Q 19/00

(54) **Ceramid-Dimere und ihre Verwendung als Arnzeimittel oder kosmetische Zubereitung**

(71) Anmelder: Wolf, Hans Uwe, 89231 Neu-Ulm (DE)
(72) Erfinder: Wolf, Hans Uwe, 89231 Neu-Ulm (DE)
(74) Vertreter: Pfenning, Meinig & Partner GbR

(57) **Zusammenfassung**

Die Erfindung betrifft Ceramid-Dimere, die aus zwei Ceramid-Molekülen aufgebaut sind, die über ihr lipophiles Ende miteinander verknüpft sind. Die Ceramid-Moleküle weisen dabei an ihrem hydrophilen Ende mindestens eine hydrophile Gruppe zur Vergrößerung der Hydrathülle des Dimers auf. Die erfindungsgemäßen Ceramid-Dimere können als Arzneimittel oder als kosmetische Zubereitung eingesetzt werden.

## Beschreibung

Die Erfindung betrifft Ceramid-Dimere, die aus zwei Ceramid-Molekülen aufgebaut sind, die über ihr lipophiles Ende miteinander verknüpft sind. Die Ceramid-Moleküle weisen dabei an ihrem hydrophilen Ende mindestens eine hydrophile Gruppe zur Vergrößerung der Hydrathülle des Dimers auf. Die erfindungsgemäßen Ceramid-Dimere können als Arzneimittel oder als kosmetische Zubereitung eingesetzt werden.

Grundsätzlich enthalten alle biologischen Membranen, insbesondere Zellmembranen, als essentielle Bestandteile sog. Lipide und Lipid-analoge Substanzen, die strukturell aus einem lipophilen (hydrophoben) und einem hydrophilen (lipophoben) Molekülteil aufgebaut sind, dass sie somit eine sog. amphiphile Struktur besitzen, die zum Teil sehr stark ausgeprägt ist.

Die amphiphile Struktur der Ceramide, d.h. die gleichzeitige Anwesenheit eines (stark) hydrophoben und eines hydrophilen, polaren Anteils der Molekülstruktur führt dazu, dass sich die Ceramide in einer wässrigen Phase (meist zusammen mit anderen Lipiden) spontan zu einer Lipid-Doppelschicht, einem so genannten "Lipid-Bilayer" anordnen, der u. a. die Grundlage der Struktur biologischer Membranen darstellt. Das Bauprinzip dieser Doppelschicht ist für Ceramide und alle anderen Lipide und Lipid-analogen Substanzen gleich: Sie ordnen sich in zwei parallelen, eng zusammenliegenden Schichten an, wobei sich jeweils die hydrophoben Reste der Ceramide direkt gegenüberliegen und in Kontakt treten. Sie bilden somit den hydrophoben inneren Bereich der Membrandoppelschicht, während die hydrophilen Reste auf beiden Seiten der Lipid-Doppelschicht mit der wässrigen Phase des Extra- und des Intrazellularraumes in Kontakt stehen. Die Tendenz zur Ausbildung dieser Ceramid-Doppelschicht besteht sowohl innerhalb als auch außerhalb eines Organismus, z. B. in einem wässrigen System, in welchem die Eigenschaften der Ceramid-Doppelschichten in eigens hierzu ausgelegten experimentellen Anordnungen untersucht werden können.

Bei allen neun bislang bekannten Ceramiden sowie bei den analogen Hydroxyceramiden besteht der lipophile Bereich aus dem Alkanrest der Sphingosin-Grundstruktur und dem an der NH-Gruppe des Sphingosins angekoppelten Fettsäurerest (Acylrest), während der hydrophile Bereich durch die beiden OH-Gruppen und durch die -NH-CO- Struktur des Sphingosin-Grundkörpers gebildet wird.

Die Struktur der Ceramid-Doppelschicht bildet sich in einem Organismus spontan aus. Obwohl sie eine erhebliche Stabilität besitzt, besteht z. B. bei Vorliegen einer Ceramid-Stoffwechselstörung die Möglichkeit, dass eine biologische Membran einen Teil ihrer Ceramid-Komponenten verliert, weil diese Komponenten entweder zu langsam und/oder in einem unzureichenden Ausmaß gebildet oder zu schnell verstoffwechselt werden. Dadurch verarmen die betreffenden Membranen an den jeweiligen Ceramid-Komponenten, was u. a. zu einer Störung der Membranstruktur und -funktion führt.

Die physiologische Zusammensetzung der Membran-Ceramide des Stratum corneum der menschlichen Haut ist allerdings noch aus einem zweiten Grund für die normale Struktur und Funktion der Haut von essentieller Bedeutung. Die Anwesenheit eines ausreichenden Gehalts an Ceramiden gewährleistet die uneingeschränkte Fähigkeit der Haut zur Bindung einer physiologischen Menge an Wasser. Der Verlust eines Teils der Stratum-corneum-Ceramide führt daher zu einer Einschränkung der Wasserbindefähigkeit, was durch die sog. Corneometrie klinisch feststellbar ist. Weiterhin erhöht sich im Zuge des Ceramidverlustes der sog. Transepidermale Wasserverlust (Trans Epidermal Water Loss = TEWL) der Haut. Dies zeigt sich im Auftreten einer "trockenen" und faltigen Haut, die insb. häufig, aber nicht ausschließlich, im höheren Lebensalter auftritt.

Ein bekanntes Beispiel für derartige Veränderungen ist die Verarmung der Lipid-Doppelschichten des Stratum corneum der menschlichen Haut an Ceramiden. Beispielsweise zeigten sich im Fall der Atopischen Dermatitis in der Haut der Patienten erniedrigte Gehalte u.a. an Ceramid 3, Ceramid 4, ω-Hydroxyceramiden (Macheleidt O, Kaiser HW und Sandhoff K (2002): J Invest Dermatol 119(1): 166-173) und Sphingosin.

Hautveränderungen und Hauterkrankungen als Folge des Verlustes von Lipiden, insb. von Ceramiden, sind beispielsweise:
- Atopische Dermatitis
- "trockene" Haut-Xerose, Xerodermie
- dyshidrotisches Ekzem
- chronisches kumulativ-toxisches Kontaktekzem
- alternde Haut
- durch UV-Licht stark beanspruchte Haut
- Sebostase
- Verhornungsstörungen
- Diabetes-bedingte Hautschäden

Insbesondere auf dem Gebiet der Klinischen Medizin ist es in den genannten Fällen von Erkrankungen wünschenswert, die Struktur der im Organismus vorhandenen biologischen Membran, i. e. der Lipid-Doppelschichten, in geeigneter Weise zu verändern und/oder zu stabilisieren.

Gemäß neueren Erkenntnissen zum Pathomechanismus der Atopischen Dermatitis (Arikawa J, Ishibashi M, Kawashima M, Takagi Y, Ichikawa Y, Imokawa G (2002): J Invest Dermatol, 119(2): 433-439) und verwandter Erkrankungen ist die Ursache für die Anfälligkeit der Haut im Fall einer derartigen Erkrankung u. a. ein veränderter Lipidstoffwechsel bzw. reduzierter Lipid-Gehalt des Stratum corneum. Diese Veränderungen betreffen neben dem Fettsäure-Stoffwechsel u.a. auch den Ceramid-Stoffwechsel.

Die heutigen Möglichkeiten, die Symptome und Folgen der genannten Hautkrankheiten, insb. der Atopischen Dermatitis, zu lindern (von einer Heilung kann derzeit noch keine Rede sein), sind nach wie vor noch sehr begrenzt. Die topische Anwendung spezieller Glucocorticoide und immunsuppressiver Wirkstoffe ist wegen der Toxizität dieser Substanzen mit erheblichen Risiken verbunden. Bestimmte Corticoide verursachen sogar einen geradezu kontraproduktiven Effekt, indem sie zu einem Verlust an Lipiden, insb. an Ceramiden, Cholesterol und freien Fettsäuren führen.

Unter Berücksichtigung des heutigen Kenntnisstands über die Bedeutung einer physiologischen LipidZusammensetzung der Stratum-corneum-Membranen ist es folgerichtig, dass versucht wird, die im Stratum corneum vorhandenen Defizite an Membran-Lipiden durch exogene Zufuhr auszugleichen. In der Praxis versucht man daher, mit Hilfe von Salben, Cremes und dergleichen die fehlenden Lipide, insb. Ceramide, der veränderten bzw. erkrankten Haut zuzuführen. Dies erfolgt beispielsweise durch speziell hierfür formulierte Lipid-Präparate, u. a. unter Verwendung von Liposomen als Vehikel zum Transport der Lipide in die Haut. Zahlreiche Produkte mit Gehalten an Ceramiden sind zu kosmetischen Zwecken und zur Therapie der genannten Hautkrankheiten inzwischen auf dem Markt.

Die hier geschilderten therapeutischen Maßnahmen sind sicherlich als prinzipiell richtig anzusehen, da sie in logischer Weise die vorhandenen Defizite des Stratum corneum an Ceramiden auszugleichen versuchen. Die während der letzten Jahre mit diesen therapeutischen Maßnahmen gemachten Erfahrungen zeigen jedoch, dass trotz der prinzipiellen Richtigkeit des therapeutischen Ansatzes die Ergebnisse dieser kosmetischen und der Heilbehandlungen keineswegs überzeugend sind. Zum Teil ist der Erfolg der durchgeführten Maßnahmen unsicher bzw. nicht nachhaltig. Selbst dann, wenn sich ein annähernd akzeptabler Erfolg der Heilbehandlung einstellt, hat eine derartige Behandlung zumindest zwei gravierende Nachteile:
- Das Ausmaß des Heilerfolgs ist nicht so groß, dass von einer vollständigen Gesundung der erkrankten Haut gesprochen werden kann.
- Um einen einigermaßen akzeptablen Heilerfolg an der Haut über einen längeren Zeitraum zu gewährleisten, müssen die Ceramide in kurzen Zeitabständen permanent der Haut zugeführt werden, d.h. die eingesetzten Wirksubstanzen zeigen keine nachhaltige Wirksamkeit.

Beide Nachteile sind auf eine gemeinsame Ursache zurückzuführen. Die Ceramide sind keine statischen Komponenten der Haut, sondern sie sind Zwischenprodukte einer Reaktionsfolge, in der die von der Haut benötigten Ceramide z.B. durch Synthesevorgänge des Organismus oder aus der Nahrung bereitgestellt und in die biologische Membran eingebaut werden. Nach Wahrnehmung ihrer Funktion als Membranbestandteil des Stratum corneum werden sie anschließend in spezifische Abbaureaktionen des Organismus eingeschleust.

Diese Reaktionsabfolge stellt ein Fließgleichgewicht dar, in welchem eine gewisse Menge an Ceramiden durch die Wirkung bestimmter Enzyme synthetisiert und nach einer entsprechenden Verweildauer in der Membran aus dieser wieder freigesetzt wird, um dann über enzymgesteuerte metabolische Abbauprozesse eliminiert zu werden. Es liegt somit ein gewisser Durchsatz an Substanz vor. Die als Hauttherapeutica exogen zugeführten Ceramide werden in diese Reaktionsabfolge eingeschleust. Liegt *a priori* eine Störung dieser Reaktionsabfolge vor, die dann zu einer pathologisch verminderten Ceramid-Zusammensetzung des Stratum corneum führt, so ist zu erwarten, dass die exogene Zufuhr von Ceramiden in Form eines Therapeuticums an diesem pathologischen Zustand grundlegend nichts oder nicht viel ändern kann, da der exogen zugeführte Ceramid-Anteil vom Organismus in gleicher Weise metabolisiert wird, wie dies bei dem endogen zur Verfügung gestellten Ceramid-Anteil der Fall ist.

Ein Heilerfolg ist daher mit den derzeit zur Verfügung stehenden therapeutischen Möglichkeiten in hohem Maße davon abhängig, dass die therapeutisch zugeführten Ceramide schneller in die Haut eindringen können als sie in die vorhandenen physiologischen Abbauschritte eingeschleust werden, und dass sie über einen längeren Zeitraum, im Extremfall lebenslang, kontinuierlich zugeführt werden.

Das vorliegende Problem ist nicht ohne weiteres lösbar. Der Geschwindigkeit der Aufnahme von Ceramiden in das Stratum corneum hinein sind gewisse physiologische und physikalisch-chemische bzw. biochemische Grenzen gesetzt, z. B. hinsichtlich der Diffusionsgeschwindigkeit der Ceramide. Diese Geschwindigkeit kann nicht beliebig gesteigert werden. Zum anderen sind die an den genannten Reaktionsfolgen beteiligten Ceramid-synthetisierenden und Ceramid-metabolisierenden Enzyme durch exogene Maßnahmen nicht oder nicht ohne gravierende Probleme im Sinne einer Erhöhung (synthetisierende Enzyme) oder einer Minderung ihrer Aktivität (metabolisierende Enzyme) zu beeinflussen.

Der erstgenannte therapeutische Ansatz, i.e., die Aktivierung lipidsynthetisierender Enzyme durch exogene Wirkstoffe (z.B. Nicotinamid), ist nur in begrenztem Umfang möglich und bisher lediglich *in vitro* gelungen (Tanno O, Ota Y, Kitamura N, Katsube T, Inoue S (2000): British J Dermatol, 143(3): 524-531). Der zweite therapeutische Ansatz, i.e., die Hemmung lipid-metabolisierender Enzyme durch exogene Wirkstoffe, ist bisher offenbar noch nicht gelungen, da offensichtlich Hemmstoffe Lipid-metabolisierender Enzyme mit ausreichend hoher Spezifität nicht existieren.

Zur Lösung des beschriebenen Problems ist es erforderlich, grundsätzlich andere Prinzipien anzuwenden, um die therapeutische Wirksamkeit exogen zugeführter Lipid-Ersatzsubstanzen oder -Analogsubstanzen zu erhöhen.

Die Aufgabe der vorliegenden Erfindung ist es deshalb, Verbindungen bereitzustellen, durch welche
- die im Organismus vorhandenen biologischen Membranen stabilisiert werden können,
- die Wasserbindefähigkeit der verschiedenen Hautschichten erhöht werden kann und
- der transepidermale Wasserverlust (TEWL = Trans Epidermal Water Loss) der Haut vermindert werden kann.

Unter der Stabilisierung der biologischen Membran soll im vorliegenden Fall der Vorgang verstanden werden, dass die erfindungsgemäßen Wirksubstanzen bereitgestellt werden, die nach Einlagerung in die biologische Membran gegenüber aus dem Stand der Technik bekannten Ceramiden eine geringere Tendenz zum Verlassen der Membran zeigen, d.h. eine größere Nachhaltigkeit ihrer kosmetischen und klinischen Wirksamkeit besitzen.

Diese Aufgabe wird durch die Ceramid-Dimere mit den Merkmalen des Anspruchs 1 sowie hinsichtlich der Verwendung als Arzneimittel mit den Merkmalen des Anspruchs 11 oder 12 sowie hinsichtlich der Verwendung als kosmetische Zubereitung mit den Merkmalen des Anspruchs 14 gelöst. Die weiteren abhängigen Ansprüche zeigen vorteilhafte Weiterbildungen auf.

Erfindungsgemäß werden Ceramid-Dimere bereitgestellt, die aus zwei Ceramid-Molekülen aufgebaut sind, die über ihr lipophiles Ende miteinander verknüpft sind.

Dabei ist es unerheblich, ob die Bindung über die Alkylgruppe des Sphingosingrundkörpers oder über den Fettsäurerest oder über beide Gruppen bzw. Reste erfolgt.

Die Ceramid-Dimere weisen dabei jeweils am hydrophilen Ende mindestens eine hydrophile Gruppe zur Vergrößerung der Hydrathülle des Dimers auf.

Die erfindungsgemäßen Verbindungen aus der Gruppe der Ceramid-Dimere weisen folgende Eigenschaften auf:
- Die grundsätzliche Struktur der eingesetzten Ceramide, welche die Ausbildung einer Lipid-Doppelmembran ermöglicht, bleibt nicht nur erhalten, sondern die Fähigkeit zur Ausbildung der Doppelmembran wird verstärkt, weil die durch die genannten Erkrankungen geschädigte Haut ohnehin nur eine eingeschränkte Fähigkeit zum Aufbau und zum Erhalt der physiologischen Lipid-Doppelmembran besitzt.
- Die Struktur der eingesetzten Ceramide wird bei erhaltener Grundstruktur so verändert, dass sie nur noch in geringerem Umfang als Substrate der in der Haut, speziell im Stratum corneum, vorhandenen metabolisierenden Enzyme fungieren können. Dies bedeutet, dass sie in deutlich geringerem Umfang als die originären Ceramide in die jeweiligen enzymatischen Reaktionsfolgen eingeschleust werden und somit als wesentliche strukturelle Bestandteile des Stratum corneum über einen längeren Zeitraum als die originären Ceramide erhalten bleiben.
- Die Abänderung der speziellen Ceramid-Molekülstruktur erfolgt jedoch andererseits nur in einem so geringen Umfang, dass durch den geringen stoffwechselbedingten Um- oder Abbau der zugeführten Ceramide nur solche Substanzen entstehen, die den körpereigenen Ceramiden möglichst ähnlich sind. Auf diese Weise wird die Gefahr erheblich verringert, dass Stoffwechselprodukte mit toxischer Wirkung entstehen.
- Eine vergleichsweise geringe, steuerbare Abbaubarkeit der Ceramid-Dimere wird dadurch erreicht, dass die kovalente Bindung zwischen den beiden originären Ceramidmolekülen über ein Sauerstoffatom erfolgt. Dieses Sauerstoffatom wirkt als metabolische Sollbruchstelle, da z.B. mischfunktionelle Monooxygenasen die in unmittelbarer Nachbarschaft zum Sauerstoffatom stehenden Kohlenstoffatome oxidativ angreifen, was zu einem Auseinanderbrechen des Ceramid-Dimers unter Bildung der ω-hydroxylierten oder carboxylierten originären Ceramide führt.
- Durch die Anbindung eines weiteren Moleküls mit ausgeprägten hydrophilen Eigenschaften an den hydrophilen Molekülteil der Ceramide wird die Hydrathülle der Ceramid-Dimere vergrößert, was in der Folge zur Erhöhung der Wasserbindefähigkeit der betreffenden Hautschichten und zu einer Verminderung des transepidermalen Wasserverlustes (TEWL) führt.

Diese Eigenschaften werden erfindungsgemäß dabei folgendermaßen erreicht.

Durch die Kopplung zweier Ceramidmoleküle zu Ceramid-Dimeren ist die Gewähr gegeben, dass ein derartiges Molekül durch die im Organismus vorhandenen Enzyme des Ceramid-Stoffwechsels sehr viel langsamer ab- bzw. umgebaut wird, als dies für die originären Ceramide zutrifft. Die mit der kovalenten Bindung zwischen zwei Ceramidmolekülen verbundene Molekülvergrößerung führt zu einer starken Minderung der enzymatisch gesteuerten Metabolisierung, weil bei der bekanntermaßen hohen Substratspezifität der meisten Enzyme die (annähernde) Verdopplung der Größe des Ceramidmoleküls zu einem Ceramid-Dimer die Geschwindigkeit des Ceramidumsatzes bzw. des Ceramidabbaus erheblich abfallen lässt.

Andererseits sind die entstehenden Abbauprodukte von ihrem allgemeinen Aufbau her den natürlicherweise vorkommenden Folgeprodukten des Ceramid-Stoffwechsels so ähnlich, dass ein Einschleusen in die entsprechenden Reaktionsfolgen nach einem langsam ablaufenden Abbau der erfindungsgemäßen Wirksubstanzen ohne Probleme möglich ist. Darüber hinaus ist auch in keiner Weise damit zu rechnen, dass die Ceramid-Dimeren wegen der großen Ähnlichkeit mit den originären Ceramiden eine relevante Toxizität besitzen.

Ein gewisser Grad der enzymatischen Metabolisierung der Ceramid-Dimere, die allerdings als deutlich geringer anzusehen ist als die der monomeren Ceramide, ist somit eine aus pharmakokinetischen und pharmakodynamischen Gründen erwünschte Eigenschaft der Wirksubstanzen, weil hierdurch die Steuerbarkeit der Therapie besser gewährleistet ist, als wenn kein metabolischer Abbau möglich wäre.

Eine gewisse kontrollierte Metabolisierungsgeschwindigkeit der Ceramid-Dimere lässt sich dadurch erreichen, dass zwischen den beiden aneinandergekoppelten Ceramiden ein Sauerstoffatom vorhanden ist. Die in Nachbarschaft zu diesem Sauerstoffatom stehenden Kohlenstoffatome können enzymatisch hydroxyliert werden, etwa durch die Cytochrom-P₄₅₀-abhängigen mischfunktionellen Monooxygenasen. Eine derartige, in unmittelbarer Nachbarschaft zum Sauerstoffatom stattfindende Hydroxylierung führt zur Bildung instabiler Verbindungen mit Halbacetalstruktur, die in die entsprechenden Reaktionsprodukte zerfallen. Das eine Reaktionsprodukt ist ein Ceramidmolekül mit ω-ständiger OH-Gruppe, das andere Reaktionsprodukt ist ein Ceramidmolekül mit ω-ständiger Aldehydfunktion, die zur Carbonsäuregruppe weiteroxidiert wird. Damit wird offensichtlich, dass durch einen oxidativ ablaufenden biochemischen Abbau der beschriebenen Ceramid-Dimere Reaktionsprodukte entstehen, die den Ausgangsverbindungen der monomeren Ceramide sehr ähnlich sind.

Ein weiterer wesentlicher Aspekt der Pathogenese der oben genannten Hautveränderungen bzw. Hauterkrankungen ist die reduzierte Wasserbindefähigkeit des Hautgewebes, insbesondere im Bereich des Stratum corneum. Physiologischerweise wird das Wasser nicht innerhalb, sondern, da mehrere parallel angeordnete Lipid-Schichten vorliegen, in den Raum zwischen den einzelnen Lipid-Doppelschichten eingelagert. Dies liegt in der Tatsache begründet, dass das Innere der Lipid-Doppelschicht aus den stark hydrophoben Fettsäureresten z.B. der Ceramide aufgebaut ist, während das Medium außerhalb der Lipid-Doppelschicht hydrophiler Natur ist. Eine Speicherung von Wasser in den hydrophoben inneren Bereichen der Lipid-Doppelmembran ist praktisch nicht möglich.

Die anfangs genannten Hautveränderungen und -erkrankungen sind zum einen auf den Verlust eines Teiles der Lipide, insb. der Ceramide, aus den parallel angeordneten Lipid-Doppelschichten, zum anderen auf den partiellen Verlust von Wasser aus den zwischen diesen Doppelschichten angeordneten hydrophilen Zwischenschichten zurückzuführen. Ziel der therapeutischen Maßnahmen bei diesen Erkrankungen ist somit nicht nur der Wiederaufbau und die Stabilisierung der Lipid-Doppelschichten selbst, wie dies mit Hilfe der oben beschriebenen Ceramid-Dimere erfolgt, sondern weiterhin auch der Aufbau und die Stabilisierung einer ausgeprägten Hydrosphäre an der Oberfläche der Lipid-Doppelschicht, die für die Wasserbindefähigkeit der Haut von ausschlaggebender Bedeutung sind.

Zwar wird eine gewisse Menge Wasser an den Lipid-Doppelschichten gebunden und trägt somit zum natürlichen Wasserreservoir der Haut bei. Bei der alternden oder erkrankten Haut reicht diese Wasserbindefähigkeit jedoch offensichtlich nicht mehr aus, um die natürliche Struktur der Haut zu erhalten. Es ist daher erforderlich, diese Wasserbindungskapazität der Lipid-Doppelschichten dadurch zu erhöhen, dass an den hydrophilen Molekülteilen der beschriebenen Ceramid-Dimere zusätzlich hydrophile Gruppen angekoppelt werden.

Dabei ist es unerheblich, ob die Bindung über die Hydroxylgruppe oder die Hydroxymethylengruppe des Sphingosingrundkörpers erfolgt.

Hydrophile Gruppen mit einer gesteigerten Tendenz zur Bindung von Wasser sind stark polare Verbindung, insb. mit positiven oder negativen Ladungen und/oder mit solchen funktionellen Gruppen, die Wasser über Wasserstoffbrückenbindungen anzulagern vermögen.
Hierzu gehören insbesondere

Aminosäuren, die stark polare Gruppen oder Ladungen besitzen: -COOH, -NH₂, -COO⁻ und -NH₃⁺, bevorzugt Serin, Threonin, Lysin, Arginin, Histidin, Asparaginsäure, Glutaminsäure, Asparagin, Glutamin, Tyrosin und Tryptophan.

Polyole, wie Ethandiol oder Glycerin (Propantriol) mit mehreren -OH-Gruppen im Molekül, die zur Ausbildung von Wasserstoffbrückenbindungen befähigt sind.

Zucker wie Glucose oder Galaktose, bei denen mehrere OH-Gruppen im Molekül vorhanden sind.

Zucker-Derivate, wie Glucuronsäure oder Galakturonsäure mit mehreren OH-Gruppen und einer dissoziierbaren -COOH-Gruppe

Zucker-Derivate, wie Aminozucker, die Bestandteile der stark wasserbindenden Hyaluronsäure darstellen.

Organische Säuren, wie die Di- bzw. Tricarbonsäuren Malonsäure, Bernsteinsäure, Äpfelsäure oder Citronensäure, deren nicht für die Bindung an das Lipidmolekül gebrauchten weiteren Carboxylreste dissoziiert und daher geladen vorliegen, was mit einer hohen Wasserbindefähigkeit einhergeht.

Anorganische Säuren, wie z.B. Sulfat und Phosphat, die auf Grund ihrer im Molekül vorhandenen Ladungen eine sehr hohe Wasserbindefähigkeit besitzen. Cholesterolsulfat kommt beispielsweise als natürlicher Bestandteil biologischer Membranen vor.

Cholin als physiologische Substanz mit einem (positiv geladenen) quarternären N-Atom.

Derivate des Harnstoffs: Diese Verbindung (auch im Deutschen unter der englischen Bezeichnung "urea" geführt) ist in freier Form bereits heute Bestandteil vieler Salben, mit denen eine Erhöhung der Wasserbindefähigkeit der Haut bewirkt werden soll.

Vorzugsweise weisen die Ceramid-Moleküle die allgemeinen Formeln I bis III auf der Basis des Sphingosins, des 6-Hydroxysphingosins und des Phytosphingosins auf:
Sphingosin-Grundkörper 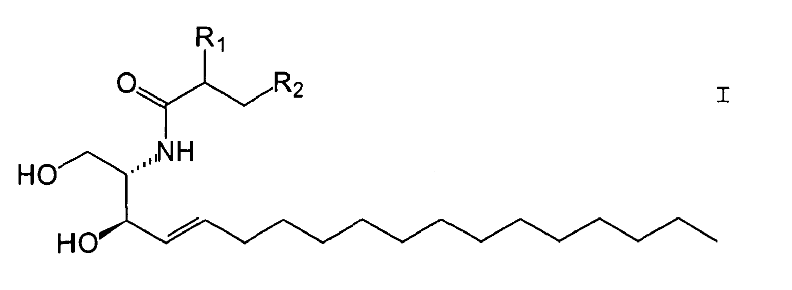
Hydroxysphingosin-Grundkörper 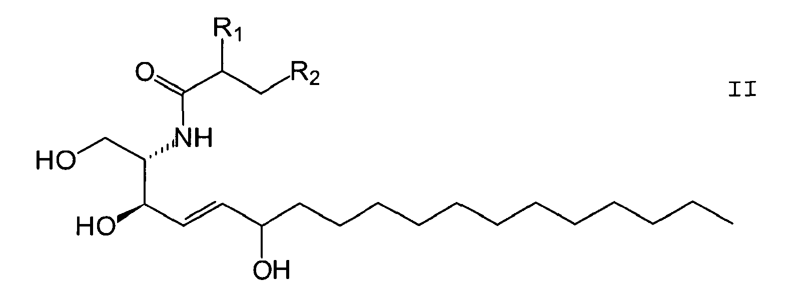
Phytosphingosin-Grundkörper 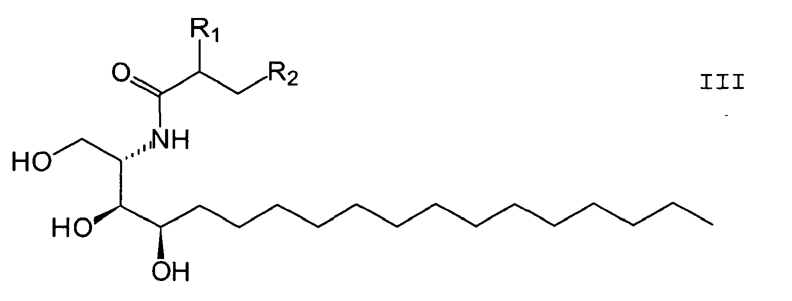
jeweils mit R₁ = H oder OH und R₂ = verzweigter oder geradkettiger C₁-C₁₆-Alkylrest, der gegebenenfalls mit Heteroatomen substituiert sein kann, oder ω-Hydroxyalkylrest mit Linolensäure verestert.

Die hierfür eingesetzten Ceramide bestehen dabei bevorzugt aus den bisher in der wissenschaftlichen Literatur beschriebenen Verbindungen Ceramid-1 (Ceramid EOS), Ceramid-2 (Ceramid NS), Ceramid-3 (Ceramid NP), Ceramid-4 (Ceramid EOH), Ceramid-5 (Ceramid AS), Ceramid-6 (Ceramid AP), Ceramid-7 (Ceramid AH), Ceramid-8 (Ceramid NH) und Ceramid-9 (Ceramid EOP) sowie den entsprechenden ω-Hydroxyceramiden. Die genannten Ceramide stellen allerdings keine einheitlichen Substanzen mit genau definierter relativer Molmasse dar, sondern jedes Ceramid stellt eine Familie von Verbindungen mit unterschiedlicher Länge der im Molekül vorhandenen amidartig gebundenen Fettsäure und/oder des im Sphingosinanteil vorhandenen Alkylrestes dar.

Die Ceramid-Moleküle sind vorzugsweise über ihr lipophiles Ende kovalent miteinander verbunden. Dabei können die Ceramid-Moleküle auch über einen Spacer verbunden sein. Der Spacer besteht dabei vorzugsweise aus mindestens einem Atom ausgewählt aus der Gruppe bestehend aus Kohlenstoff, Stickstoff oder Sauerstoff oder Kombinationen hiervon, vorzugsweise bestehend aus einem Sauerstoffatom oder einer -O-(CH₂)ₙ-O-Gruppe mit n = 1 bis 20._Ebenso ist es möglich, dass der Spacer eine der genannten Gruppen enthält.

Erfindungsgemäß wird ebenso das zuvor beschriebene Ceramid-Dimer zur Verwendung als Arzneimittel bereitgestellt.

Erfindungsgemäß wird das Ceramid-Dimer, wie es zuvor beschrieben wurde, zur Herstellung eines Arzneimittels, zur Behandlung von Erkrankungen, bei denen eine Störung der Lipidzusammensetzung der Zellmembranen eines Organismus hinsichtlich ihres Gehalts an Ceramiden und Sphingosinen vorliegt, bereitgestellt.

Das zuvor beschriebene Ceramid-Dimer kann auch zur Herstellung eines Arzneimittels zur Behandlung von Erkrankungen, bei denen eine Störung der Zusammensetzung der Lipid-Doppelschichten des Stratum corneum der Haut hinsichtlich ihres Gehaltes an Ceramiden und Sphingosinen vorliegt, eingesetzt werden.

Eine weitere Verwendung der erfindungsgemäßen Ceramid-Dimere betrifft die Herstellung von kosmetischen Zubereitungen, insbesondere als Creme, Salbe, Lotion, Emulsion, Gel, Spray, kosmetisches Öl oder Liposomen.

Anhand der nachfolgenden Figuren und Beispiele soll der erfindungsgemäße Gegenstand näher erläutert werden, ohne diesen auf die hier gezeigten speziellen Ausführungsformen einschränken zu wollen.

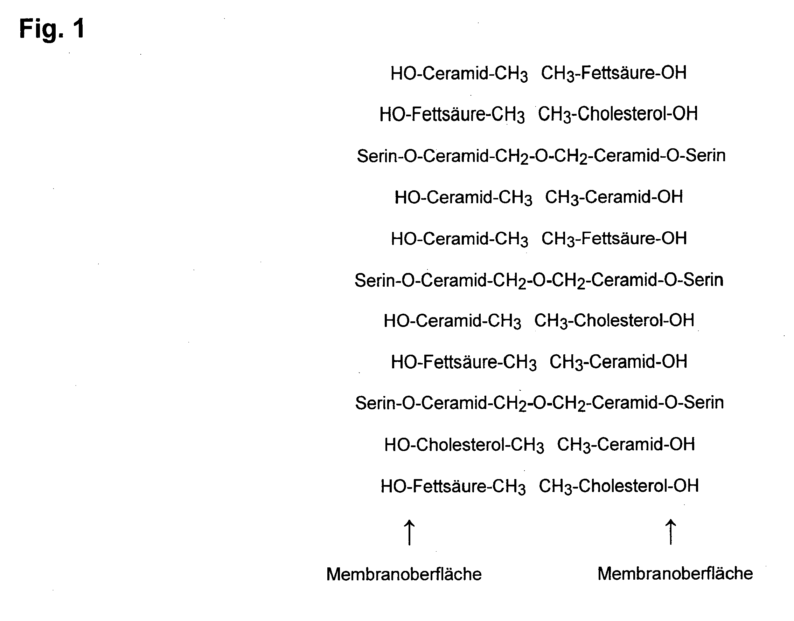

Fig. 1 zeigt den schematischen Aufbau biologischer Membranen des Stratum corneum aus den Hauptlipiden Ceramide, Cholesterol und freien Fettsäuren mit der zusätzlichen Einlagerung von Ceramid-Dimeren mit zwei angekoppelten Serin-Resten.
Fig. 2 zeigt eine erfindungsgemäße Wirksubstanz, in der die Lipid-Komponente aus einem Dimer des Ceramid 2 besteht.
Fig. 3 zeigt eine erfindungsgemäße Wirksubstanz, in der die Lipid-Komponente aus einem Dimer des Ceramid 2 besteht. Zur Erhöhung der Hydrophilie der an der Membranoberfläche liegenden hydrophilen Molekülareale ist an einer der beiden Ceramid-2-Komponenten ein Lysinrest angekoppelt.
Fig. 4 zeigt eine erfindungsgemäße Wirksubstanz, in der die Lipid-Komponente aus einem Dimer des Ceramid 2 besteht. Zur Erhöhung der Hydrophilie der an der Membranoberfläche liegenden hydrophilen Molekülareale ist an den beiden Ceramid-2-Komponenten jeweils ein Serinmolekül angekoppelt.
Fig. 5 zeigt eine erfindungsgemäße Wirksubstanz, in der die Lipid-Komponente aus einem Molekül Ceramid 2 und einem Molekül Ceramid 5 besteht. Zur Erhöhung der Hydrophilie der an der Membranoberfläche liegenden hydrophilen Molekülareale sind an den beiden Ceramid-Komponenten ein Glutaminsäurerest und ein Asparaginsäurerest angekoppelt.

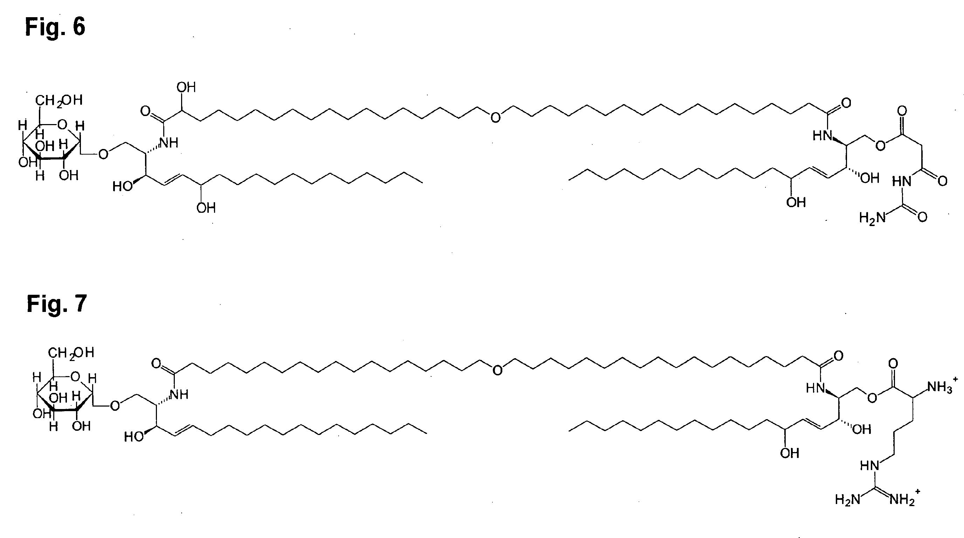

Fig. 6 zeigt eine erfindungsgemäße Wirksubstanz, in der die Lipid-Komponente aus einem Molekül Ceramid 7 und einem Molekül Ceramid 8 besteht. Zur Erhöhung der Hydrophilie der an der Membranoberfläche liegenden hydrophilen Molekülareale ist am Ceramid 7 ein Glucoserest und am Ceramid 8 ein Harnstoffderivat (einen Malonsäurerest enthaltend) angekoppelt.
Fig. 7 zeigt eine erfindungsgemäße Wirksubstanz, in der die Lipid-Komponente aus einem Molekül Ceramid 2 und einem Molekül Ceramid 8 besteht. Zur Erhöhung der Hydrophilie der an der Membranoberfläche liegenden hydrophilen Molekülareale ist am Ceramid 2 ein Glucoserest und am Ceramid 8 ein Arginin-Rest angekoppelt.

### Beispiel

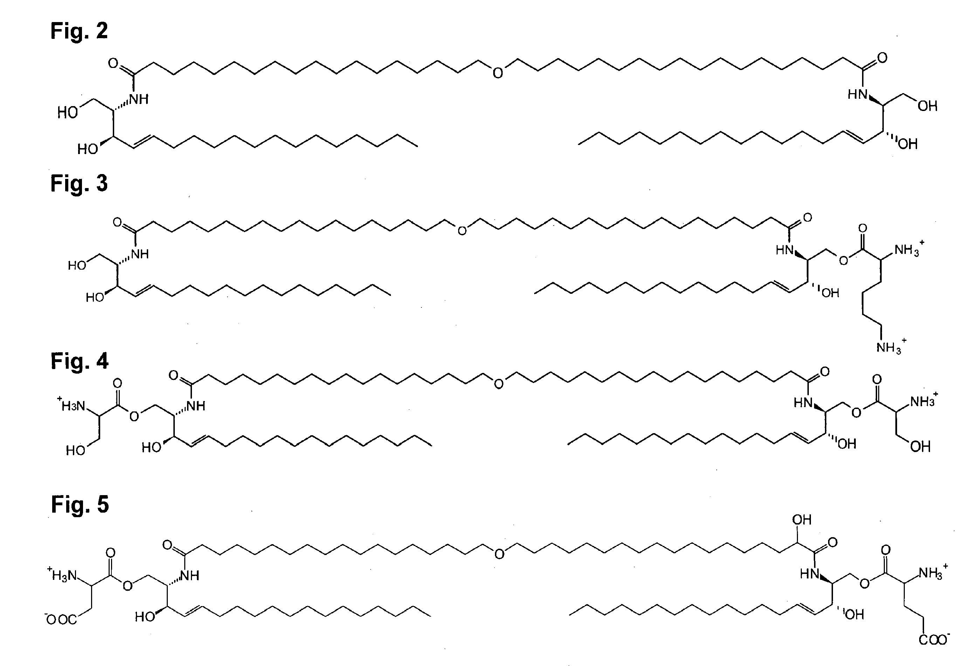

Gemäß den fachärztlichen dermatologischen Gutachten der Dermatest(R) GmbH, 48143 Münster, zeigen die beiden erfindungsgemäßen Wirkstoffe 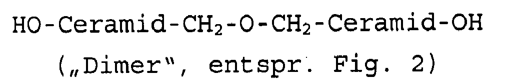 und 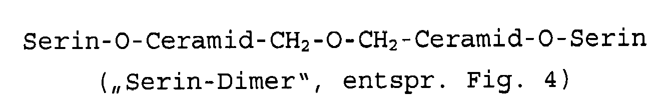 mit Ceramid-2 als Ceramidkomponente hinsichtlich der Corneometrie (Wasserbindevermögen) und TEWL (Wasserverlust) bei 10 Probanden mit Neurodermitis und 10 Probanden mit Xerodermie nach 1, 2 und 4 Wochen die folgenden Ergebnisse (alle Angaben in % Veränderung gegenüber dem Ausgangswert):
**Dimer** (entspr. Fig. 2):
**Corneometrie** (Wasserbindevermögen):

| | 1 Woche | 2 Wochen | 4 Wochen |
|---|---|---|---|
| | | | |
| Alle 20 Probanden | + 24,05 | + 28,02 | + 37,60 |
| 10 Prob. Neurodermitis | + 25,63 | + 28,62 | + 43,40 |
| 10 Prob. Xerodermie | + 22,52 | + 27,42 | + 31,90 |

**TEWL** (Wasserverlust):

| | 1 Woche | 2 Wochen | 4 Wochen |
|---|---|---|---|
| | | | |
| Alle 20 Probanden | - 8,65 | - 13,32 | - 16,75 |
| 10 Prob. Neurodermitis | - 7,81 | - 13,50 | - 17,02 |
| 10 Prob. Xerodermie | - 9,46 | - 13,04 | - 16,39 |

Beim TEWL-Wert wird die Minderung des Wasserverlustes erfasst. Dies bedeutet, dass die Wirksamkeit der erfindungsgemäßen Wirksubstanz umso höher ist, je stärker negativ der Messwert ist.
**Serin-Dimer** (s. Fig. 4):
**Corneometrie** (Wasserbindevermögen):

| | 1 Woche | 2 Wochen | 4 Wochen |
|---|---|---|---|
| | | | |
| Alle 20 Probanden | + 25,43 | + 29,49 | + 40,87 |
| 10 Prob. Neurodermitis | + 27,71 | + 32,88 | + 47,43 |
| 10 Prob. Xerodermie | + 23,23 | + 26,21 | + 34,53 |

**TEWL** (Wasserverlust):

| | 1 Woche | 2 Wochen | 4 Wochen |
|---|---|---|---|
| | | | |
| Alle 20 Probanden | - 7,80 | - 12,57 | - 15,37 |
| 10 Prob. Neurodermitis | - 6,20 | - 9,74 | - 12,84 |
| 10 Prob. Xerodermie | - 9,36 | - 15,45 | - 17,93 |

Die Ergebnisse der durchgeführten dermatologischen Untersuchungen besagen, dass beide erfindungsgemäßen Wirksubstanzen eine gute bis sehr gute Wirksamkeit hinsichtlich der (erwünschten) Erhöhung des Wasserbindevermögens der Haut und hinsichtlich der (erwünschten) Erniedrigung des Wasserverlustes aus der Haut besitzen.

Eine differenziertere Bewertung der Ergebnisse zeigt, dass das Serin-Dimer stärker als das Dimer das Wasserbindevermögen der Haut begünstigt. Dieser Effekt tritt besonders beim Vorliegen von Neurodermitis auf.

Andererseits begünstigt das Dimer etwas stärker als das Serin-Dimer den Wasserverlust aus der Haut im Fall der Neurodermitis.

## Patentansprüche

1. Ceramid-Dimer aus zwei Ceramid-Molekülen, die über ihr lipophiles Ende miteinander verknüpft sind, **dadurch gekennzeichnet, dass** am hydrophilen Ende der Ceramid-Moleküle mindestens eine hydrophile Gruppe zur Vergrößerung der Hydrathülle des Dimers angeordnet ist.

2. Ceramid-Dimer nach Anspruch 1,
**dadurch gekennzeichnet, dass** die hydrophile Gruppe ausgewählt ist aus der Gruppe bestehend aus
Aminosäuren, insbesondere Aminosäuren mit polaren Gruppen oder Ladungen, bevorzugt ausgewählt aus der Gruppe bestehend aus -COOH, -NH₂, -COO⁻ und -NH₃⁺, bevorzugt Serin, Threonin, Lysin, Arginin, Histidin, Asparaginsäure, Glutaminsäure, Asparagin, Glutamin, Tyrosin und Tryptophan.
Polyole, insbesondere Ethandiol oder Glycerin, Zucker, insbesondere Glucose oder Galaktose, Zuckerderivate mit einer dissoziierbaren -COOH-Gruppe, insbesondere Glucuronsäure oder Galakturonsäure, oder Aminozucker,
organische Säuren, insbesondere Di- bzw. Tricarbonsäuren, bevorzugt Malonsäure, Bernsteinsäure, Äpfelsäure oder Citronensäure,
anorganischen Säuren, insbesondere Schwefel- oder Phosphorsäuren,
Cholin,
Harnstoff oder Harnstoffderivate
sowie Kombinationen hiervon.

3. Ceramid-Dimer nach einem der vorhergehenden Ansprüche,
**dadurch gekennzeichnet, dass** die Bindung der hydrophilen Gruppe entweder über die Hydroxylgruppe oder die Hydroxymethylengruppe des Sphingosingrundkörpers erfolgt.

4. Ceramid-Dimer nach dem vorhergehenden Anspruch, **dadurch gekennzeichnet, dass** die Ceramide einen Sphingosin-Grundkörper, Hydroxysphingosin-Grundkörper oder einen Phytosphingosin-Grundkörper gemäß einer der allgemeinen Formeln I bis III aufweisen: 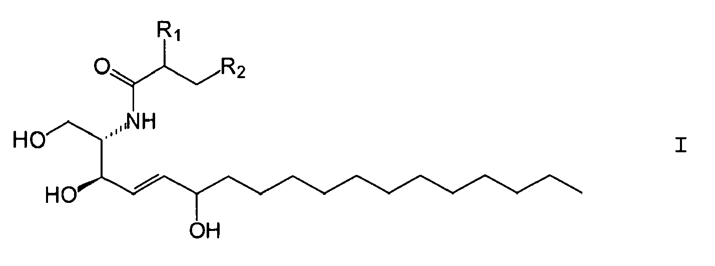 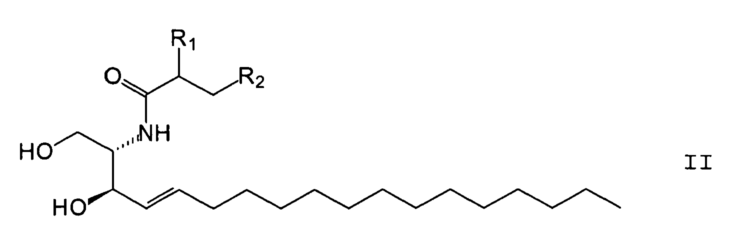 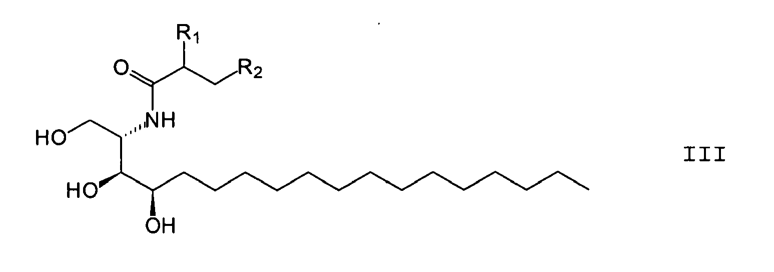 jeweils mit R₁ = H oder OH und R₂ = verzweigter oder geradkettiger C₁-C₁₆-Alkylrest, der gegebenenfalls mit Heteroatomen substituiert sein kann, oder ω-Hydroxyalkylrest mit Linolensäure verestert.

5. Ceramid-Dimer nach dem vorhergehenden Anspruch, **dadurch gekennzeichnet, dass** die Ceramid-Moleküle ausgewählt sind aus der Gruppe bestehend aus Ceramid-1, Ceramid-2, Ceramid-3, Ceramid-4, Ceramid-5, Ceramid-6, Ceramid-7, Ceramid-8, Ceramid-9 sowie deren ω-Hydroxyceramiden.

6. Ceramid-Dimer nach einem der vorhergehenden Ansprüche,
**dadurch gekennzeichnet, dass** die Ceramid-Moleküle über ihr lipophiles Ende kovalent miteinander verbunden sind.

7. Ceramid-Dimer nach einem der vorhergehenden Ansprüche,
**dadurch gekennzeichnet, dass** die kovalente Bindung der Ceramid-Moleküle über ihr lipophiles Ende entweder über die Alkylkette der Sphingosin-Grundstruktur oder über den Fettsäurerest oder über die Kombination beider Angriffspunkte erfolgt.

8. Ceramid-Dimer nach einem der vorhergehenden Ansprüche,
**dadurch gekennzeichnet, dass** die Ceramid-Moleküle über einen Spacer verbunden sind.

9. Ceramid-Dimer nach dem vorhergehenden Anspruch, **dadurch gekennzeichnet, dass** der Spacer aus mindestens einem Atom ausgewählt aus der Gruppe bestehend aus Kohlenstoff, Stickstoff oder Sauerstoff oder Kombinationen hiervon besteht oder diese enthält.

10. Ceramid-Dimer nach dem vorhergehenden Anspruch, **dadurch gekennzeichnet, dass** der Spacer vorzugsweise aus einem Sauerstoffatom oder einer -O-(CH₂)ₙ-O-Gruppe mit n = 1 bis 20 besteht.

11. Ceramid-Dimer nach einem der vorhergehenden Ansprüche zur Verwendung als Arzneimittel.

12. Ceramid-Dimer nach einem der Ansprüche 1 bis 10 zur Herstellung eines Arzneimittels zur Behandlung von Erkrankungen, bei denen eine Störung der Lipidzusammensetzung der Zellmembranen eines Organismus hinsichtlich ihres Gehalts an Ceramiden, ω-Hydroxyceramiden und Sphingosinen vorliegt.

13. Ceramid-Dimer nach einem der Ansprüche 1 bis 10 zur Herstellung eines Arzneimittels zur Behandlung von Erkrankungen, bei denen eine Störung der Zusammensetzung der Lipid-Doppelschichten des Stratum corneum der Haut hinsichtlich ihres Gehaltes an Ceramiden, ω-Hydroxyceramiden und Sphingosinen vorliegt.

14. Verwendung der Ceramid-Dimere nach einem der Ansprüche 1 bis 10 zur Herstellung einer kosmetischen Zubereitung, insbesondere als Creme, Salbe, Lotion, Emulsion, Gel, Spray, kosmetisches Öl oder Liposomen.
